# NEW EUROPEAN PATENT SPECIFICATION

(11) **EP 0 610 511 B2**
(45) Date of publication and mention of the opposition decision: **01.08.2007**
(45) Mention of the grant of the patent: 02.04.2003
(21) Application number: 93914996.9
(22) Date of filing: 13.07.1993
(51) Int. Cl.: A61K 8/45, A61K 8/00, A61K 31/57, A61K 31/19, A61K 31/80, A61K 31/70, A61K 31/60, A61K 31/07, A61K 31/405, A61K 31/195, A61K 31/165

(54) **COMPOSITION FOR DERMATOLOGIC PREPARATION**
ZUSAMMENSETZUNG FÜR DERMATOLOGISCHE ZUBEREITUNG
COMPOSITION POUR PREPARATION DERMATOLOGIQUE

(30) Priority: 13.07.1992 JP 22772392; 13.07.1992 JP 22772492; 13.07.1992 JP 22772692
(43) Date of publication of application: 17.08.1994
(73) Proprietor: SHISEIDO COMPANY, LTD., Tokyo 104-8010 (JP)
(72) Inventor: YANAGIDA, Takeshi The Shiseido Research Center, Yokohama-shi Kanagawa 223 (JP); SAKAMOTO, Okihiko The Shiseido Research Center, Yokohama-shi Kanagawa 223 (JP)
(74) Representative: Santarelli
(86) International application number: PCT/JP1993/000968
(87) International publication number: WO 1994/001083

(56) References cited:
- EP-A- 0 158 090
- EP-A- 0 255 364
- EP-A- 0 273 202
- EP-A- 0 440 398
- EP-A- 0 512 814
- WO-A-85/03434
- WO-A-86/06275
- DE-A- 1 792 514
- DE-A- 3 327 840
- DE-A- 3 431 755
- GB-A- 1 239 965
- JP-A- 1 063 031
- JP-A- 1 186 809
- JP-A- 1 186 811
- JP-A- 57 020 298
- JP-A- 57 131 716
- JP-A- 58 140 008
- JP-A- 59 095 210
- JP-A- 62 106 022
- JP-A- 63 010 710
- RO-A- 59 202
- US-A- 4 369 180
- US-A- 4 826 828
- US-A- 4 847 071
- US-A- 5 013 726
- CTFA, 7th ed., 1997

## Description

### TECHNICAL FIELD

The present invention relates to an external skin treatment composition and, more specifically, it relates to an external skin treatment composition having a synergistically improved skin roughening improvement effect by incorporating thereinto vitamin A and a polyoxyalkylene modified organopolysiloxane or an anti-inflammatory agent, with taking safety into consideration.

### BACKGROUND ART

Various pharmaceutically effective components are formulated into external skin treatment compositions. Among these pharmaceutical effects, an effect, by which the changes in the skin due to aged skin or sunlight exposure etc. are prevented or improved, is one of such effects, and therefore, an external skin treatment compositions such as cosmetic compositions having such purposes have been desired.

Under such circumstances, various raw materials extracted from natural products, such as proteins, polysaccharides, extracted extracts, natural polymers etc. have been heretofore formulated in external skin treatment compositions due to their characteristics application effects.

Recently, Japanese Unexamined Patent Publication (Kokai) No. 64-500355 discloses a method for preventing or improving the changes or disabilities caused due to aged skins or sunlight exposure by formulating thereinto at least one component selected from vitamin A and the derivatives thereof.

However, the effects thereof are not sufficient and it has been strongly desired to develop a pharmaceutically effective agent having much more excellent effects.

European patent application 0 512 814 discloses topical compositions for preventing or reducing the damaging effects of ultra-violet light on skin comprising 1-hydroxycholecalciferol and/or 1,25 dihydroxycholecalciferol in combination with a sunscreen material and optionally retinol or a derivative thereof.

German patent application DE-A-1 792 514 discloses cosmetic formulations comprising glucidamines which essentially comprise three fractions :
a) partially depolymerized glycoproteins containing hexoses, hexosamines and short chain proteins;
b) depolymerized polysaccharides consisting of hexosamines and uronic acids;
c) sulfonated polysaccharides containing -SO₃H groups, partially acetylated hexosamines and uronic acids.

These compositions are useful for treatment of cellulitis and skin dehydration, for epidemic regeneration and for the treatment of wrinkles and of the breast.

US Patent N° 5 013 726 relates to an external analgesic lotion containing as active ingredients methyl salicylate, camphor and menthol. This lotion can also comprise, among others, Vitamin A. This lotion is useful to relieve pain in muscles, joints or viscera distal at the side of application by stimulating cutaneous sensory receptors.

Japanese patent application N° 57-131716 relates to a cosmetic composition comprising sitosterol as an essential component. This composition is useful for the prevention of drying and keratinization of the plantar skin.

International application N° WO 85/03434 discloses a delivery system for pharmaceutically active ingredients to the human skin for enhanced percutaneous absorption, comprising a mixture of fatty alcohol, volatile silicone and a pharmaceutically active ingredient.

### DISCLOSURE OF THE INVENTION

Accordingly, the objects of the present invention are to obviate the above-mentioned problems in the prior art and to provide an external skin treatment composition having sufficient skin roughening improvement effects, i.e., prevention effects, improvement effects, against the changes or disabilities due to aged skin or sunlight exposure.

In accordance with the present invention, there is provided an external skin treatment composition comprising (i) vitamin A and (ii) at least one skin roughening improvement aid selected from the group consisting of (a) polyoxyalkylene modified organopolysiloxanes and (c) anti-inflammatory agents according to claim 1.

### Best Mode for Carrying out the Invention

In order to achieve the above-mentioned objects, the present inventors have been extensibly studied to obtain a substance or substances capable of effecting sufficient skin roughening improvement effects, especially, among substances having excellent safety and, as a result, found that the above-mentioned problems can be solved by formulating, together with vitamin A, polyoxyalkylene modified organopolysiloxanes or anti-inflammatory agents according to claim 1.

The constitution of the present invention will now be explained in detail.

Vitamin A used in the present invention is also called retinol and is usually used in the treatment of infant or childhood diseases or nyctalopia (i.e., night blindness) or in the recovery agent after pregnancy in the pharmaceutical fields. Among these, all-trans products or 13-cis products can be preferably used, but the mixture thereof can also be used.

The amount of vitamin A formulated into the external skin treatment agent according to the present invention, is 0.00001 to 5% by weight, more preferably 0.0001 to 0.5% by weight, in view of the effect of vitamin A to the skin.

The polyoxyalkylene modified organopolysiloxanes usable as a skin roughening improvement aid, in the present invention are the following compounds (A), (B), (C) and (D). 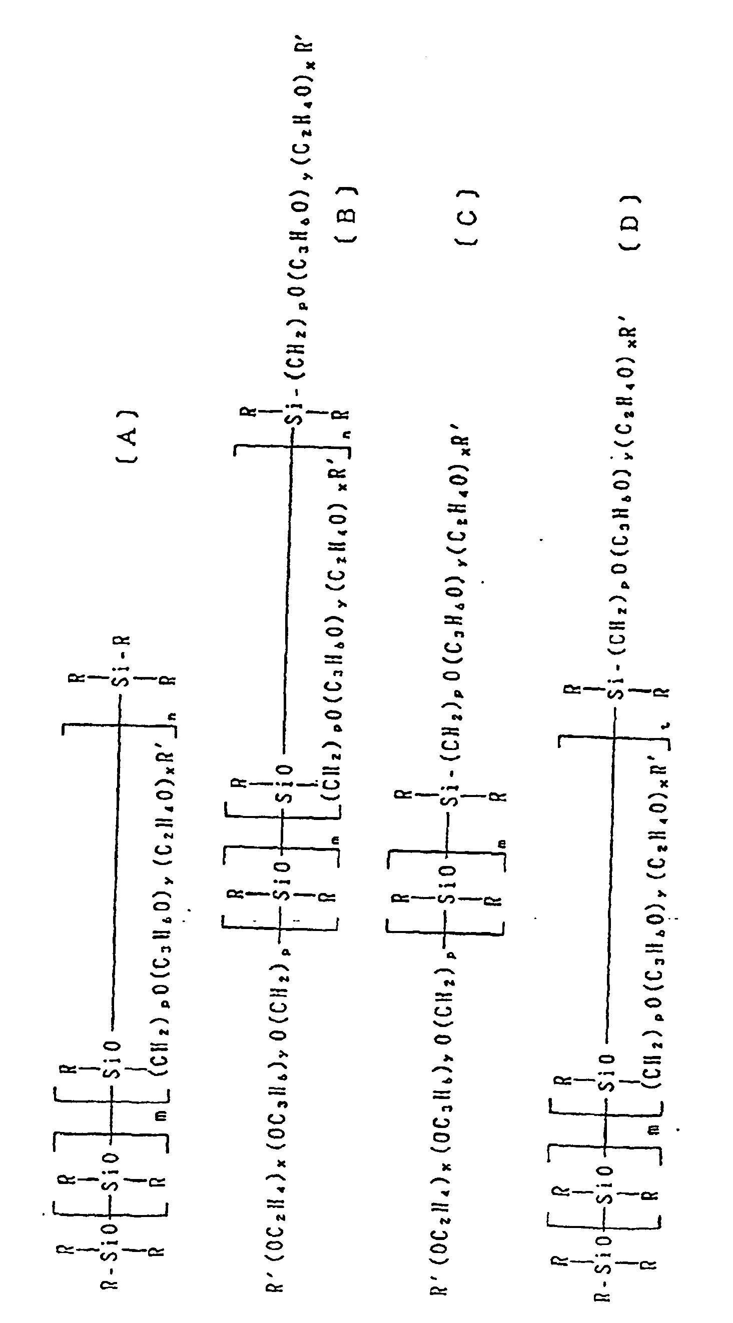 wherein R represents an alkyl group having 1 to 3 carbon atoms, or a phenyl group, R' represents hydrogen or an alkyl group having 1 to 12 carbon atoms, p is an integer of 1 to 5, m is an integer of 5 to 100, n and x are an integer of 1 to 50 and t and y are an integer of 0 to 50.

There are no specific limitations to the average molecular weight of the polyoxyalkylene modified organopolysiloxane usable in the present invention, but the preferable molecular weight is 3,000 or more, further preferably 5,000 to 10,000. Furthermore, the preferable polyoxyalkylene modified organopolysiloxanes are those having 2 - 80% by weight, more preferable, 11 - 50% by weight in view of the generation of the effects, of polyoxyalkylene group in the molecule thereof.

The amount of the polyoxyalkylene modified organopolysiloxane formulated is 0.1 to 20% by weight, preferably 0.2 to 10% by weight, in the total amount of the external skin treatment composition. When the amount is less than 0.1% by weight, there are fears that the skin irritation is not sufficiently lowered. Contrary to this, when the amount is more than 20% by weight, there are fears that the qualities necessary as the skin treatment composition cannot be held.

In the present invention, the composition further comprises sugars mention may be made of monosaccharides, oligosaccharides or sugar alcohols

As the monosaccharides, mention may be made of trioses such as D-glycerylaldehyde, dihydroxyaceton, tetroses such as D-erythrose, D-erythrolose, D-threose, pentoses such as L-arabinose, D-xylose, L-lyxose, D-arabinose, D-ribose, D-ribulose, D-xylulose, L-xylulose, hexoses such as D-glucose, D-talose, D-psicose, D-galactose, D-fructose, L-galactose, L-sorbose, D-mannose, D-tagalose, heptoses such as aldoheptose, heptalose etc., octoses such as octose, deoxy sugars such as 2-deoxy-D-ribose, 6-deoxy-L-galactose, 6-deoxy-L-mannose, amino sugars such as D-glucosamine, D-galactosamine, sialic acid, aminouronic acid, muramic acid, uronic acids such as D-glucuronic acid, D-mannuronic acid, L-gluconic acid, D-galacturonic acid, L-iduronic acid.

As the oligo sugars, mention may be made of sucrose, gentianose, umbelliferose, lactose, planteose, α,α-trehalose, raffinose, umbilicin, stachyose, verbascoses.

Furthermore, as the sugar alcohols, mention may be made of sorbitol, maltiol, maltriose, mannitol, starch decomposed sugar, erythritol, xylitose, starch decomposing sugar reduced alcohols. Of these sugar alcohols, mannitol, erythritol and sugar alcohols of disaccharides or more.

There are no specific limitations to the amount of the sugars formulated, the preferable amount is at least 0.1% by weight, more preferably 0.5 to 50% by weight, based on the total amount of the external skin treatment agent.

Examples of anti-inflammatory agents usable as the skin roughening improvement aid according to the present invention are hydrocortisone, hydrocortisone acetate, prednisolone, methyl prednisolone, prednisolone acetate, prednisolone acetate propionate, dexamethasone, betamethasone, triamcinolone, dexamethasone acetate, betamethasone valerate, triamcinolone acetnide, aspirin, salicylic acid, acetaminophen, glycol salicylate, mefenamic acid, flufenamic acid, indometacin, diclofenac, ketoprofen, ibuprofen, flurbiprofen, fenbufen, lufexamac, piroxicam, oxyphenbutazone, mepirizole, ibuprofen piconol, clidanac, phenylbutazone, naproxen, glycyrrhetin, glycyrrhizin, glycyrrhetic acid and the salts and esters thereof, glycyrrhizic acid and the salts and esters thereof, azulene, thymol, allantoin. Among these agents, one or more agents may be freely selected. The amount of the anti-inflammatory agents formulated in the present invention is 0.0001 to 5.0% by weight, preferably 0.001 to 2% by weight, based upon the total amount of the external skin treatment composition. When the amount is less than 0.0001% by weight, there are fears that the reduction of the skin irritation, which is the effect of the present external skin treatment composition, cannot be achieved. Contrary to this, even when the anti-inflammatory agent is formulated in an amount of more than 5.0% by weight, further improvement is not expected.

In addition to the above-mentioned essential components, the external skin treatment composition according to the present invention may contain various components conventionally formulated into cosmetics, quasi-drugs, drugs such as aqueous components, humectants, thickeners, UV absorbers, antiseptics, antioxidants, flavours, colorants, medicines, crude drugs, in an amount such that the desired effects of the present invention are not impaired. It should be, of course, noted that these additives are used in such quantitative, qualitative conditions that the objects of the present invention are not impaired.

The external skin treatment composition according to the present invention can be in any form, for example, in the form of a solubilized type such as cosmetic lotions, an emulsified type such as Emulsions, creams, ointments, powder dispersion type, water-oil two layer type, water-oil-powder three layer type.

### EXAMPLES

The present invention will now be further illustrated by no means limited to, the following Examples, in which the amounts formulated are "% by weight".

### Examples 1-1 to 1-5

Creams having the following compositions were prepared and the skin roughening improvement effects thereof were studied.

The polyoxyalkylene modified organopolysiloxanes listed in Table 1-1 were formulated.

| | | % |
|---|---|---|
| (1) | Cetostearyl alcohol | 3.5 |
| (2) | Squalane | 30.0 |
| (3) | Beeswax | 3.0 |
| (4) | Reduced lanolin | 5.0 |
| (5) | Ethylparaben | 0.3 |
| (6) | Polyoxyethylene (50 mol) | 2.0 |
| | oleyl alcohol ether | |
| (7) | Stearic monoglyceride | 2.0 |
| (8) | Polyoxyalkylene modified | 3.0 |
| | organopolysiloxane (see Table 1-1) | |
| (9) | Perfume | 0.03 |
| (10) | Vitamin A | 0.0001 |
| (11) | Glycerol | 15.0 |
| (12) | Purified water | Balance |

### (Preparation method)

(1), (2), (3), (4), (5), (6), (7), (8), (9) and (10) were heated and dissolved. The resultant solution was maintained at 75°C, followed by adding thereto, under stirring, (11) and (12) heated to 75°C. The mixture was stirred and emulsified in a homomixer, followed by cooling to obtain a cream.

### Comparative Example 1-1

The same formulation except that the polyoxyalkylene modified organopolysiloxane was removed from the formulation of Example 1-1.

### Comparative Example 1-2

The same formulation except that the vitamin A was removed from the formulation of Example 1-1.

**Table 1-1**

| No. | Polyoxyalkylene modified organosiloxane |
|---|---|
| Example 1-1 | General formula A Polyoxyethylene group 20 wt%, M.W. 6,000 |
| | |
| Example 1-2 | General formula A Polyoxyethylene group 40 wt%, M.W. 20,000 |
| | |
| Example 1-3 | General formula B Polyoxyethylene group 60 wt%, M.W. 10,000 |
| | |
| Example 1-4 | General formula C Polyoxyethylene group 20 wt%, Polyoxypropylene group 10 wt%, M.W. 4,000 |
| | |
| Example 1-5 | General formula A Polyoxyethylene group 15 wt%, M.W. 2,500 |

### Skin Roughening Improvement Test Method

One hundred subjects having psoriasis-like and skin-roughening-like skin affections were divided into 5 groups as test panels and the creams of Examples 1-1 to 1-5 and Comparative Examples 1-1 to 1-2 were used by one group (20 members) each. That is, Examples 1-1 to 1-5 samples were applied to the left-side faces of the panel twice a day and Comparative Examples 1-1 and 1-2 were applied to the right-side faces of the panel for continuous 3 months. Thereafter, the degree of the overall improvements after the use was visually determined, when compared before the use.

The results are shown in Table 1-2.

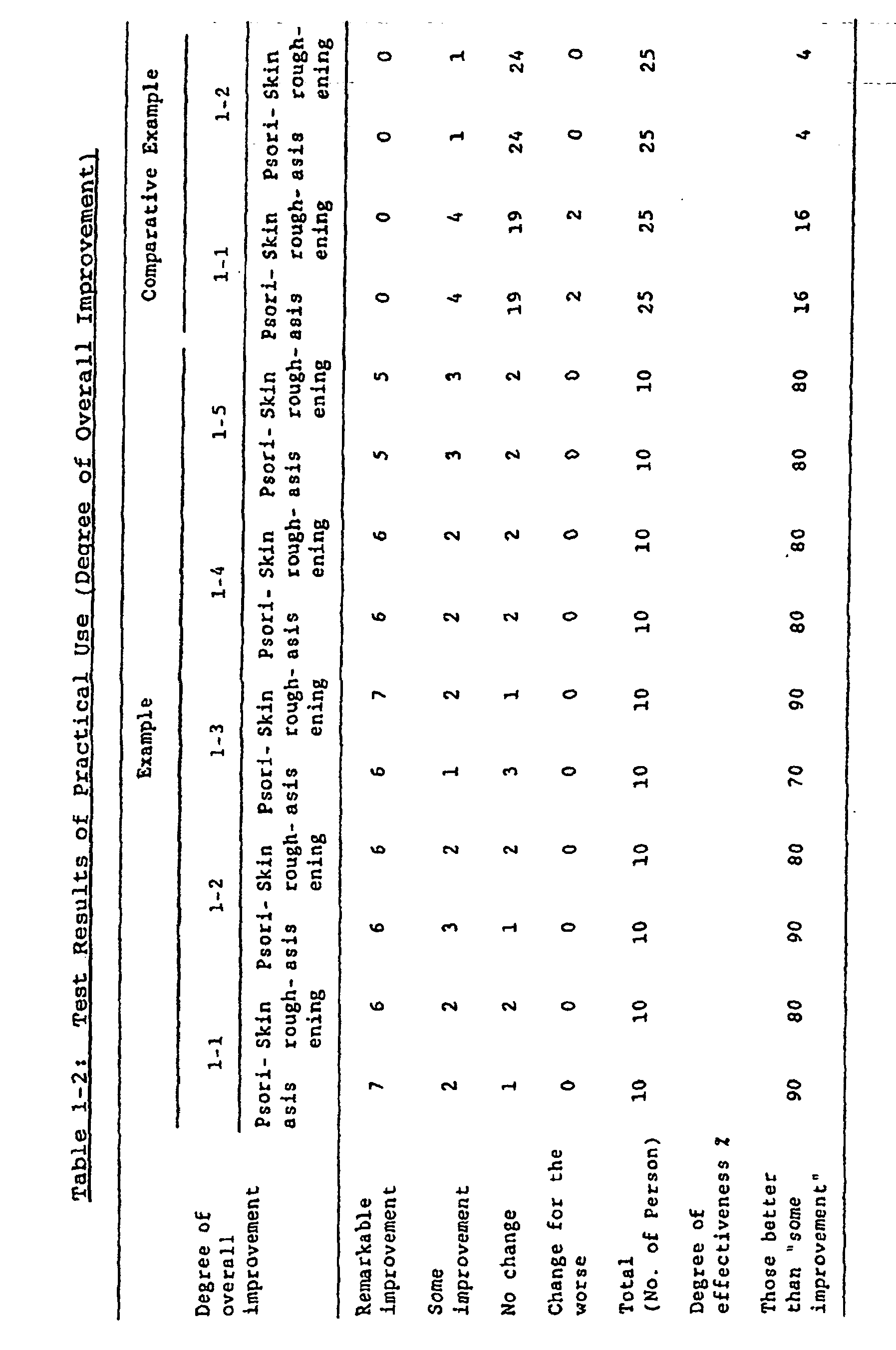

As is clear from the results shown in Table 1-2, the products of Examples 1-1 to 1-5 according to the present invention had synergistically excellent skin roughening improvement effects when compared with those of Comparative Examples 1-1 and 1-2.

| Example 1-6: Cosmetic lotion | | % |
|---|---|---|
| (1) | Vitamin A | 0.00001 |
| (2) | Polyoxyalkylene modified organopolysiloxane^{*1} | 0.1 |
| (3) | Glycerol | 1.0 |
| (4) | D-mannitol | 0.5 |
| (5) | Purified water | Balance |
| (6) | Ethanol | 7.0 |
| (7) | Polyoxyethylene (50 mol) oleyl alcohol ether | 1.0 |
| (8) | Methylparaben | 0.05 |
| (9) | Oleyl alcohol | 1.0 |
| (10) | Lactic acid | 0.01 |
| (11) | Sodium lactate | 0.1 |
| (12) | Perfume | 0.01 |

| | | |
|---|---|---|
| *1: General formula B (Polyoxyethylene group 60 wt%, M.W. 10000) | | |

### (Preparation method)

In the purified water, (3), (10) and (11) were dissolved. Separately, (1), (2), (7), (8) and (12) were dissolved in ethanol and this solution was added to the above purified water to be dissolved, followed by filtration. Thus, the cosmetic lotion was obtained. The cosmetic lotion of the present invention was excellent in the skin roughening improvement effects.

| Example 1-7: Pack | | % |
|---|---|---|
| (1) | Polyoxyalkylene modified organopolysiloxane^{*2} | 3.0 |
| (2) | Polyvinyl alcohol | 10.0 |
| (3) | Propylene glycol | 7.0 |
| (4) | Ethanol | 10.0 |
| (5) | Vitamin A | 0.01 |
| (6) | Methylparaben | 0.05 |
| (7) | POE(60 mol) hydrogenated castor oil | 0.2 |
| (8) | Perfume | 0.05 |
| (9) | Purified water | Balance |

| | | |
|---|---|---|
| *2: General formula A (Polyoxyethylene group 40 wt%, M.W. 8000) | | |

### (Preparation method)

(1), (3), (6) and (7) were added to (7) and dissolved under stirring. Then, (2) was added thereto and stirred under heating, followed by adding thereto (4) containing (9) dissolved therein. The mixture was dissolved while stirring to obtain the pack.

The present pack had excellent skin roughening improvement effects.

| Example 1-8: Compact face powder | | % |
|---|---|---|
| (1) | Vitamin A | 0.0005 |
| (2) | Talc | 85.4 |
| (3) | Stearic acid | 2.5 |
| (4) | Squalane | 3.5 |
| (5) | Sorbitan sesquioleic ester | 1.8 |
| (6) | Triethanolamine | 1.2 |
| (7) | Polyoxyalkylene modified organopolysiloxane^{*3} | 10.0 |
| (8) | Glycyrrhetic stearyl | 0.1 |
| (9) | Pigment | q.s. |
| (10) | Perfume | q.s. |

| | | |
|---|---|---|
| *3: General formula B (polyoxyethylene group 60 wt%, M.W. 4000) | | |

### (Preparation method)

The talc and the pigment were sufficiently mixed by a kneeder (Powder portion). The triethanolamine was added to 50% corresponding amount of the purified water and the mixture was maintained at 70°C (Aqueous phase). The components of the present invention other than the perfume were mixed and dissolved under heating at 70°C (Oil phase). The oil phase was added to the aqueous phase, followed by uniformly emulsified by a homomixer and the resultant emulsified mixture was added to the powder portion, followed by kneeding the same by a kneeder, followed by evaporating the water and by treating the same by a grinder. Furthermore, the perfume was uniformly sprayed and the resultant product was compression molded.

The resultant compact face powder was excellent in the improvement effects to the skin.

| Example 1-9: Lipstick | | % |
|---|---|---|
| (1) | Vitamin A | 0.00001 |
| (2) | Microcrystalline wax | 3.0 |
| (3) | Beeswax | 3.0 |
| (4) | Ceresin wax | 5.0 |
| (5) | Liquid paraffin | 19.0 |
| (6) | Squalane | 20.0 |
| (7) | Carnauba wax | 3.0 |
| (8) | Candelilla wax | 3.0 |
| (9) | Polyoxyalkylene modified organopolysiloxane^{*4} | 1.0 |
| (10) | Mixed colorant | 7.0 |
| (11) | Dibutyl hydroxytoluene | 0.05 |
| (12) | Perfume | q.s. |
| (13) | Lanolin | Balance |

| | | |
|---|---|---|
| *4: General formula A (Polyoxyethylene group 60 wt%, M.W. 25000) | | |

### (Preparation method)

The lipstick was obtained in a conventional way. The present lipstick exhibited remarkable prevention of the generation of roughening on the lips.

| Example 1-10: Emulsion | | % |
|---|---|---|
| (1) | Vitamin A | 1.0 |
| (2) | Polyoxyalkylene modified organopolysiloxane^{*5} | 1.0 |
| (3) | Ethanol | 2.0 |
| (4) | Glycerol | 10.0 |
| (5) | Sorbitol 70% solu. | 3.0 |
| (6) | Propylene glycol | 3.0 |
| (7) | Carboxyvinyl polymer | 0.3 |
| (8) | KOH | 0.1 |
| (9) | Methylparaben | 0.1 |
| (10) | Cetanol | 2.5 |
| (11) | Vaseline | 2.0 |
| (12) | Squalane | 10.0 |
| (13) | Isopropyl myristate | 5.0 |
| (14) | Glyceryl monostearate | 2.0 |
| (15) | POE(25 mol) cetyl ether | 2.0 |
| (16) | Purified water | Balance |

| | | |
|---|---|---|
| *5: General formula C (Polyoxyethylene group 15 wt%, Polyoxypropylene group 10 wt%, M.W. 5000) | | |

### (Preparation method)

The present emulsion was obtained in a conventional way. The present emulsion exhibited excellent skin improvement effects.

| Example 1-11: Emulsion | | % |
|---|---|---|
| (1) | Vitamin A | 0.3 |
| (2) | Polyoxyalkylene modified organopolysiloxane^{*6} | 0.2 |
| (3) | Ethanol | 5.0 |
| (4) | Glycerol | 5.0 |
| (5) | Sorbitol | 2.0 |
| (6) | Propylene glycol | 5.0 |
| (7) | Carboxyvinyl polymer | 0.2 |
| (8) | KOH | 0.06 |
| (9) | Methyl paraben | 0.2 |
| (10) | POE(60 mol) hydrogenated castor oil | 1.0 |
| (11) | Squalane | 3.0 |
| (12) | Isopropyl myristate | 3.0 |
| (13) | Indometacin | 0.05 |
| (14) | Purified water | Balance |

| | | |
|---|---|---|
| *6: General formula D (Polyoxyethylene group 40 wt%, M.W. 7000) | | |

### (Preparation method)

The present emulsion was obtained in a conventional way. The present emulsion exhibited excellent skin improvement effects.

| Example 1-12: Night cream | | % |
|---|---|---|
| (1) | Squalane | 20.0 |
| (2) | Liquid paraffin | 10.0 |
| (3) | Isopropyl myristate | 6.0 |
| (4) | Butyl paraben | 0.2 |
| (5) | Polyoxyalkylene modified organopolysiloxane^{*7} | 3.0 |
| (6) | Diglycerol diisostearate | 1.0 |
| (7) | Vaseline | 4.0 |
| (8) | Solid paraffin | 2.0 |
| (9) | Vitamin A | 0.3 |
| (10) | Propylene glycol | 4.0 |
| (11) | Glycerol | 10.0 |
| (12) | Magnesium sulfate | 0.3 |
| (13) | Purified water | Balance |

| | | |
|---|---|---|
| *7: General formula A (Polyoxyethylene group 20 wt%, M.W. 6000) | | |

### (Preparation method)

The present night cream was obtained in a conventional way. The present night cream exhibited excellent skin improvement effects.

| Example 2-6: Cosmetic lotion | | % |
|---|---|---|
| (1) | Vitamin A | 0.1 |
| (2) | D-xylose | 0.1 |
| (3) | Glycerol | 1.0 |
| (4) | Purified water | Balance |
| (5) | Ethanol | 7.0 |
| (6) | Polyoxyethylene (20 mol) oleyl alcohol ether | 0.5 |
| (7) | Methyl paraben | 0.05 |
| (8) | Citric acid | 0.01 |
| (9) | Sodium citrate | 0.1 |
| (10) | Camphor | 0.01 |
| (11) | Perfume | 0.01 |

### (Preparation method)

In the purified water, (2), (3), (8) and (9) were dissolved. Separately, (1), (6), (7) and (11) were dissolved in ethanol and this solution was added to the above purified water to be dissolved, followed by filtration. Thus, the cosmetic lotion was obtained. The cosmetic lotion of the present invention was excellent in the skin roughening improvement effects.

| Example 2-12: Emulsion | | % |
|---|---|---|
| (1) | Vitamin A | 0.3 |
| (2) | L-arabinose | 2.5 |
| (3) | Ethanol | 5.0 |
| (4) | Glycerol | 5.0 |
| (5) | Propylene glycol | 5.0 |
| (6) | Carboxyvinyl polymer | 0.2 |
| (7) | KOH | 0.06 |
| (8) | Methyl paraben | 0.2 |
| (9) | POE(60 mol) hydrogenated castor oil | 1.0 |
| (10) | Squalane | 3.0 |
| (11) | Isopropyl myristate | 3.0 |
| (12) | Monoammonium glycyrrhizinate | 0.05 |
| (13) | Purified water | Balance |

### (Preparation method)

The present emulsion was obtained in a conventional way. The present emulsion exhibited synergistically excellent skin improvement effects.

### Examples 3-1 to 3-5

Creams having the following compositions were prepared and the skin roughening improvement effects thereof were studied. The anti-inflammatory agents formulated are listed in Table 3-1.

| | | % |
|---|---|---|
| (1) | Cetostearyl alcohol | 3.5 |
| (2) | Squalane | 30.0 |
| (3) | Beeswax | 3.0 |
| (4) | Reduced lanolin | 5.0 |
| (5) | Ethyl paraben | 0.3 |
| (6) | Polyoxyethylene (50 mol) oleyl alcohol ether | 2.0 |
| (7) | Stearic monoglyceride | 2.0 |
| (8) | Inflammatory agent (see Table 3-1) | 0.1 |
| (9) | Perfume | 0.03 |
| (10) | Vitamin A | 0.0001 |
| (11) | Glycerol | 15.0 |
| (12) | Purified water | Balance |

### (Preparation method)

(1), (2), (3), (4), (5), (6), (7), (8), (9) and (10) were heated and dissolved. The resultant solution was maintained at 75°C, followed by adding thereto, under stirring, (11) and (12) heated to 75°C. The mixture was stirred and emulsified in a homomixer, followed by cooling to obtain a cream.

### Comparative Example 3-1

The same formulation except that the anti-inflammatory agents was removed from the formulation of Example 3-1.

### Comparative Example 3-2

The same formulation except that the vitamin A was removed from the formulation of Example 3-1.

**Table 3-1**

| | Drugs formulated |
|---|---|
| Example 3-1 | Glycyrrhizic ammonium |
| Example 3-3 | Glycyrrhetic stearyl |
| Example 3-4 | Hydrocortisone |
| Example 3-5 | Acetaminophen |

### Skin Roughening Improvement Test Method

One hundred subjects having psoriasis-like and skin-roughening-like skin affections were divided into 5 groups as test panels and the creams of Examples 3-1 to 3-5 and Comparative Examples 3-1 to 3-2 were used by one group (20 members) each. That is, Examples 3-1 to 3-5 samples were applied to the left-side faces of the panel twice a day and Comparative Examples 3-1 and 3-2 were applied to the right-side faces of the panel for continuous 3 months. Thereafter, the degree of the overall improvements after the use was visually determined, when compared before the use.

The results are shown in Table 3-2.

**Table 3-2: Test Results of Practical Use (Degree of Overall Improvement)**

| Degree of overall improvement | Example | | | | | | | | Comparative Example | | | |
|---|---|---|---|---|---|---|---|---|---|---|---|---|
| | 3-1 | | 3-3 | | 3-4 | | 3-5 | | 3-1 | | 3-2 | |
| | Psoriasis | Skin roughening | Psoriasis | Skin roughening | Psoriasis | Skin roughening | Psoriasis | Skin roughening | Psoriasis | Skin roughening | Psoriasis | Skin roughening |
| Remarkable improvement | 7 | 6 | 6 | 7 | 6 | 6 | 5 | 5 | 0 | 0 | 0 | 0 |
| | | | | | | | | | | | | |
| Some improvement. | 2 | 2 | 1 | 2 | 2 | 2 | 3 | 3 | 4 | 4 | 3 | 3 |
| | | | | | | | | | | | | |
| No change | 1 | 2 | 3 | 1 | 2 | 2 | 2 | 2 | 19 | 19 | 22 | 22 |
| | | | | | | | | | | | | |
| Change for the worse | 0 | 0 | 0 | 0 | 0 | 0 | 0 | 0 | 2 | 2 | 0 | 0 |
| | | | | | | | | | | | | |
| Total (No. of Person) | 10 | 10 | 10 | 10 | 10 | 10 | 10 | 10 | 25 | 25 | 25 | 25 |
| | | | | | | | | | | | | |
| Degree of effectiveness % | | | | | | | | | | | | |
| | | | | | | | | | | | | |
| Those better than "some improvement" | 90 | 80 | 70 | 90 | 80 | 80 | 80 | 80 | 16 | 16 | 12 | 12 |

As is clear from the results shown in Table 3-2, the products of the present invention of Examples 3-1 to 3-5 had the synergistically excellent skin roughening improvement effects, when compared with the products of Comparative Examples 3-1 and 3-2.

| Example 3-6: Cosmetic lotion | | % |
|---|---|---|
| (1) | Vitamin A | 0.00001 |
| (2) | Bethamethasone | 0.01 |
| (3) | Glycerol | 1.0 |
| (4) | Maltitol | 0.3 |
| (5) | Purified water | Balance |
| (6) | Ethanol | 7.0 |
| (7) | Polyoxyethylene (50 mol) oleyl alcohol ether | 1.0 |
| (8) | Methyl paraben | 0.05 |
| (9) | Oleyl alcohol | 1.0 |
| (10) | Lactic acid | 0.01 |
| (11) | Sodium lactate | 0.1 |
| (12) | Perfume | 0.01 |

### (Preparation method)

In the purified water, (3), (4), (10) and (11) were dissolved. Separately, (1), (2), (7), (8) and (12) were dissolved in ethanol and this solution was added to the above purified water to be dissolved, followed by filtration. Thus, the cosmetic lotion was obtained. The cosmetic lotion of the present invention was excellent in the skin roughening improvement effects.

| Example 3-7: Pack | | % |
|---|---|---|
| (1) | Dexamethasone acetate | 0.5 |
| (2) | Polyvinyl alcohol | 10.0 |
| (3) | Propylene glycol | 7.0 |
| (4) | Ethanol | 10.0 |
| (5) | Vitamin A | 0.01 |
| (6) | Methyl paraben | 0.05 |
| (7) | POE(60 mol) hydrogenated castor oil | 0.2 |
| (8) | Perfume | 0.05 |
| (9) | Purified water | Balance |

### (Preparation method)

In (9), (1), (3), (6) and (7) were added, followed by stirring, whereby the mixture was dissolved. Then, (2) was added thereto and the mixture was stirred under heating. Then, (4) containing (9) dissolved therein was added and stirred, whereby the mixture was dissolved to obtain the pack.

The present pack exhibited the excellent skin roughening improvement effects.

| Example 3-8: Compact face powder | | % |
|---|---|---|
| (1) | Vitamin A | 0.0005 |
| (2) | Talc | 85.4 |
| (3) | Stearic acid | 2.5 |
| (4) | Squalane | 3.5 |
| (5) | Sorbitan sesquioleic ester | 1.8 |
| (6) | Triethanolamine | 1.2 |
| (7) | Diclofenac | 0.01 |
| (8) | Pigment | q.s. |
| (9) | Perfume | q.s. |

### (Preparation method)

The talc and the pigment were sufficiently mixed by a kneeder (Powder portion). The triethanolamine was added to 50% corresponding amount of the purified water and the mixture was maintained at 70°C (Aqueous phase). The components of the present invention other than the perfume were mixed and dissolved under heating at 70°C (Oil phase). The oil phase was added to the aqueous phase, followed by uniformly emulsified by a homomixer and the resultant emulsified mixture was added to the powder portion, followed by kneeding the same by a kneeder, followed by evaporating the water and by treating the same by a grinder. Furthermore, the perfume was uniformly sprayed and the resultant product was compression molded.

The resultant compact face powder was excellent in the improvement effects to the skin.

The present compact face powder exhibited excellent skin improvement effects.

| Example 3-9: Lipstick | | % |
|---|---|---|
| (1) | Vitamin A | 0.00001 |
| (2) | Microcrystalline wax | 3.0 |
| (3) | Beeswax | 3.0 |
| (4) | Ceresin wax | 5.0 |
| (5) | Liquid paraffin | 19.0 |
| (6) | Squalane | 20.0 |
| (7) | Carnauba wax | 3.0 |
| (8) | Candellira wax | 3.0 |
| (9) | Glycyrrhizinic stearyl | 5.0 |
| (10) | Mixed colorant | 7.0 |
| (11) | Dibutyl hydroxytoluene | 0.05 |
| (12) | Perfume | q.s. |
| (13) | Lanolin | Balance |

### (Preparation method)

The lipstick was obtained in a conventional way. The present lipstick prevented the formation of roughening on the lip.

| Example 3-10: Emulsion | | % |
|---|---|---|
| (1) | Vitamin A | 1.0 |
| (2) | Hydrocortisone acetate | 0.05 |
| (3) | Ethanol | 2.0 |
| (4) | Glycerol | 10.0 |
| (5) | Mannitol | 3.0 |
| (6) | Propylene glycol | 3.0 |
| (7) | Carboxyvinyl polymer | 0.3 |
| (8) | KOH | 0.1 |
| (9) | Methyl paraben | 0.1 |
| (10) | Cetanol | 2.5 |
| (11) | Vaseline | 2.0 |
| (12) | Squalane | 10.0 |
| (13) | Isopropyl myristate | 5.0 |
| (14) | Glyceryl monostearate | 2.0 |
| (15) | POE(25 mol) cetyl ether | 2.0 |
| (16) | Purified water | Balance |

### (Preparation method)

The present emulsion was obtained in a conventional way. The present emulsion exhibited excellent skin improvement effects.

| Example 3-11: Emulsion | | % |
|---|---|---|
| (1) | Vitamin A | 0.3 |
| (2) | Indometacin | 0.3 |
| (3) | Ethanol | 5.0 |
| (4) | Glycerol | 5.0 |
| (5) | Propylene glycol | 5.0 |
| (6) | Carboxyvinyl polymer | 0.2 |
| (7) | KOH | 0.06 |
| (8) | Methyl paraben | 0.2 |
| (9) | POE(60 mol) hydrogenated castor oil | 1.0 |
| (10) | Squalane | 3.0 |
| (11) | Isopropyl myristate | 3.0 |
| (12) | Purified water | Balance |

### (Preparation method)

The present emulsion was obtained in a conventional way. The present emulsion exhibited excellent skin improvement effects.

| Example 3-12: Night cream | | % |
|---|---|---|
| (1) | Squalane | 10.0 |
| (2) | Liquid paraffin | 10.0 |
| (3) | Vaseline | 3.0 |
| (4) | Cetyl octanoate | 10.0 |
| (5) | Dibutyl phthalate | 5.0 |
| (6) | Glycyrrhizinic stearyl | 0.1 |
| (7) | Indometacin | 0.2 |
| (8) | Butyl paraben | 0.2 |
| (9) | Diglycerine triisostearate | 2.0 |
| (10) | Diglycerine monoisostearate | 1.5 |
| (11) | Vitamin A | 0.1 |
| (12) | Glycerol | 10.0 |
| (13) | Propylene glycol | 6.0 |
| (14) | Purified water | Balance |

### (Preparation method)

The present night cream was obtained in a conventional way. The present night cream exhibited excellent skin improvement effects.

### [Industrial Applicability]

The external skin treatment composition according to the present invention are useful as an external skin treatment composition capable of preventing the epidermal disabilities and synergistically of improving the changes and disabilities due to aged skins or sunlight exposure, with taking safety into consideration.

## Claims

1. An external skin treatment composition comprising (i) 0.00001 to 5.0% by weight of Vitamin A and (ii) at least one skin roughening improvement aid selected from the group consisting of (a) 0.1 to 20% by weight of polyoxyalkylene modified organopolysiloxanes, and (c) 0.0001 to 5.0% by weight of anti-inflammatory agents, said polyoxyalkylene modified organopolysiloxanes are selected from the group consisting of the compounds represented by the formulae (A), (B), (C) and (D) herebelow, wherein R represents an alkyl group having 1 to 3 carbon atoms or a phenyl group, R' is hydrogen or an alkyl group having 1 to 12 carbon atoms, p is an integer of 1 to 5, m is an integer of 5 to 100, n and x are an integer of 1 to 50, and t and y are an integer of 0 to 50 : 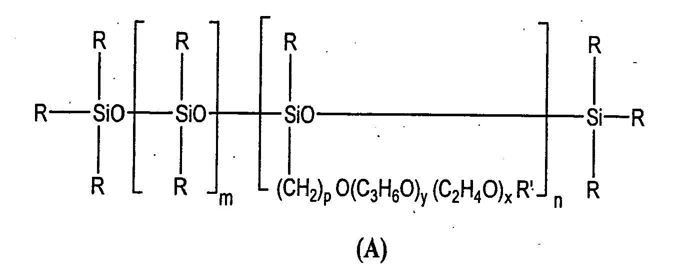 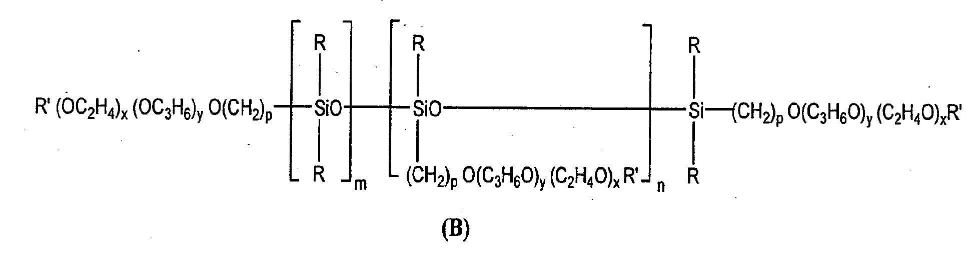 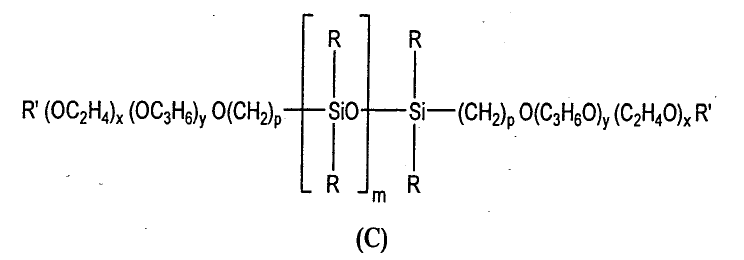 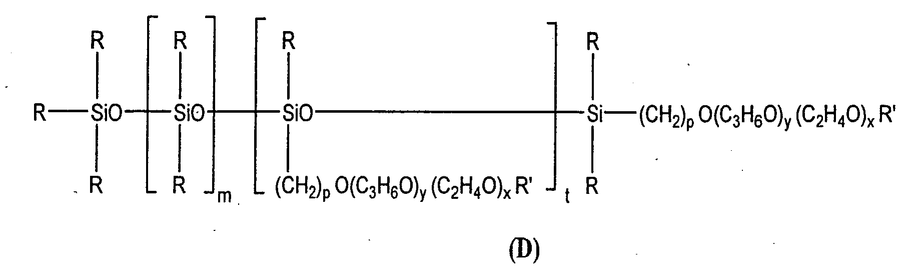 said anti-inflammatory agents are selected from the group consisting of hydrocortisone, hydrocortisone acetate, prednisolone, methyl prednisolone, prednisolone acetate, prednisolone acetate propionate, dexamethasone, betamethasone, triamcinolone, dexamethasone acetate, betamethasone valerate, triamcinolone acetonide, aspirin, acetaminophen, glycol salicylate, mefenamic acid, flufenamic acid, indometacin, diclofenac, ketoprofen, ibuprofen, flurbiprofen, fenbufen, lufexamac, piroxicam, oxyphenbutazone, mepirizole, ibuprofen piconol, clidanac, phenylbutazone, naproxen, glycyrrhetin, glycyrrhizin, glycyrrhetic acid and the salts and esters thereof, glycyrrhizic acid and the salts and esters thereof, azulene and thymol.

2. Composition according to claim 1, which further comprises at least one sugar.

3. Composition according to claim 2, wherein the sugar is selected from the group consisting of monosaccharides, oligosaccharides and sugar alcohols.

4. Composition according to one of claims 2 and 3, wherein the amount of the sugar formulated in the composition is 0.1 % by weight or more.

5. Use of an external skin treatment composition comprising (i) 0.00001 to 5.0 % by weight of vitamin A and (ii) at least one skin roughening improvement aid selected from the group consisting of (a) 0.1 to 20% polyoxyalkylene modified organopolysiloxanes, and (c) 0.0001 to 5.0% anti-inflammatory agents as agent for improving or alleviating skin roughening.

6. Use according to claim 5, wherein the skin roughening improvement aid is at least one polyoxyalkylene modified organopolysiloxane, selected from the group consisting of the compounds represented by the formulae (A), (B), (C) and (D) below, wherein R represents an alkyl group having 1 to 3 carbon atoms or a phenyl group, R' is hydrogen or an alkyl group having 1 to 12 carbon atoms, p is an integer of 1 to 5, m is an integer of 5 to 100, n and x are an integer of 1 to 50, and t and y are an integer of 0 to 50 :

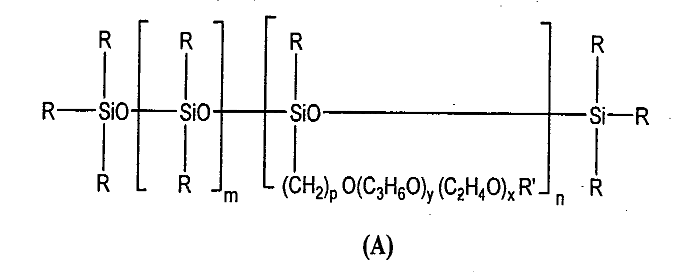

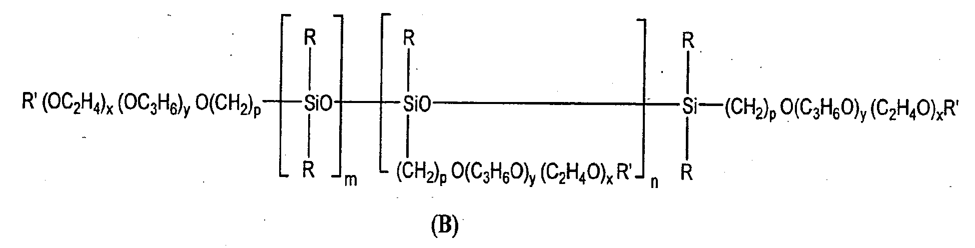

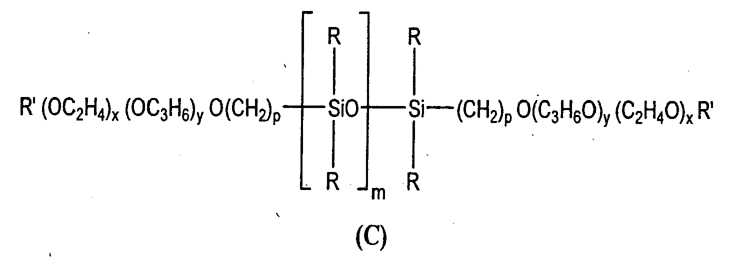

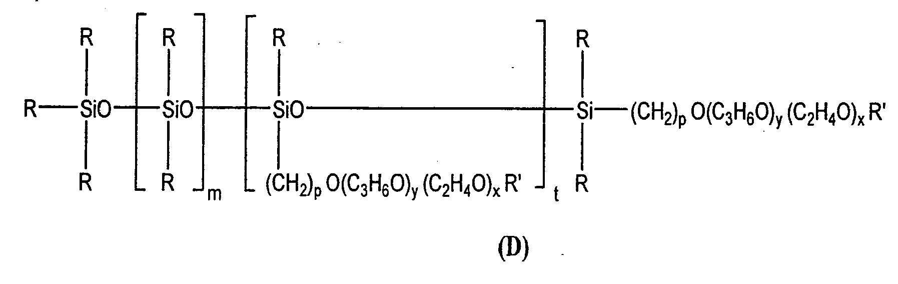

7. Use according to claims 5 or 6, wherein the skin roughening improvement aid is at least one anti-inflammatory agent selected from the group consisting of hydrocortisone, hydrocortisone acetate, prednisolone, methyl prednisolone, prednisolone acetate, prednisolone acetate propionate, dexamethasone, betamethasone, triamcinolone, dexamethasone acetate, betamethasone valerate, triamcinolone acetonide, aspirin, salicylic acid, acetaminophen, methyl salicylate, glycol salicylate, mefenamic acid, flufenamic acid, indometacin, diclofenac, ketoprofen, ibuprofen, flurbiprofen, fenbufen, lufexamac, piroxicam, oxyphenbutazone, mepirizole, ibuprofen piconol, clidanac, phenylbutazone, naproxen, glycyrrhetin, glycyrrhizin, glycyrrhetic acid and the salts and esters thereof, glycyrrhizic acid and the salts and esters thereof, azulene, camphor, thymol and allantoin.

8. Use according to any one of claims to claims 5 to 7, wherein said external skin treatment composition further comprises at least one sugar, preferably a sugar selected from the group consisting of monosaccharides, oligosaccharides and sugar alcohols.

## Patentansprüche

1. Eine Zusammensetzung für die äußerliche Behandlung der Haut, welche (i) 0,00001 bis 5,0 Gew.-% Vitamin A und (ii) wenigstens ein Hilfsmittel zur Verbesserung der Hautrauheit, das ausgewählt ist aus der Gruppe, bestehend aus (a) 0,1 bis 20 Gew.-% Polyoxyalkylen-modifizierten Organopolysiloxanen und (c) 0,0001 bis 5,0 Gew.-% entzündungshemmenden Mitteln umfasst, wobei die Polyoxyalkylen-modifizierten Organopolysiloxane ausgewählt sind aus der Gruppe, bestehend aus den Verbindungen, welche durch die nachstehenden Formeln (A), (B), (C) und (D) dargestellt sind, worin R für eine Alkylgruppe mit 1 bis 3 Kohlenstoffatomen oder eine Phenylgruppe steht, R' Wasserstoff oder eine Alkylgruppe mit 1 bis 12 Kohlenstoffatomen ist, p eine ganze Zahl von 1 bis 5 ist, m eine ganze Zahl von 5 bis 100 ist, n und x ganze Zahlen von 1 bis 50 sind, und t und y ganze Zahlen von 0 bis 50 sind; 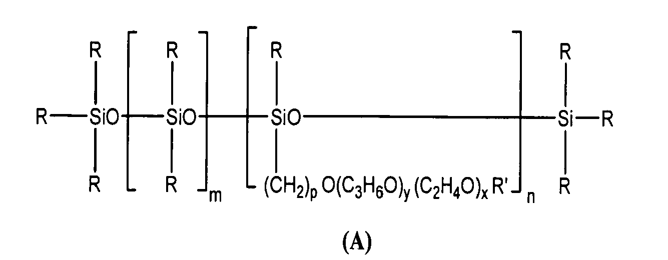 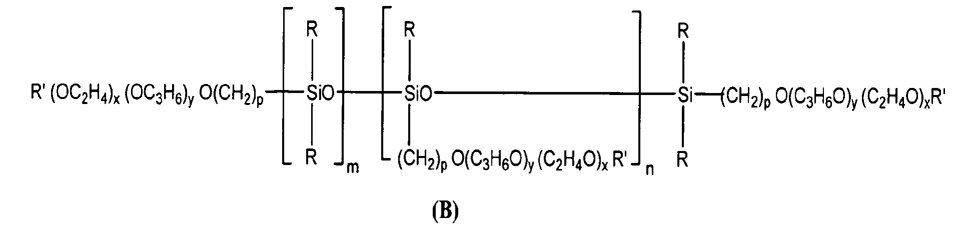 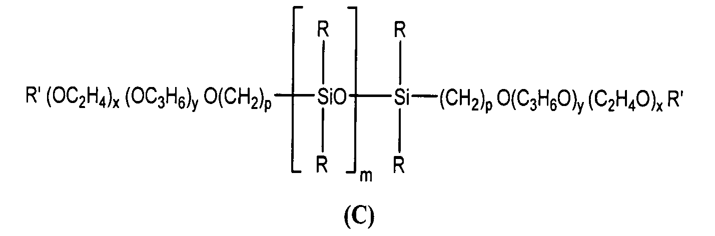 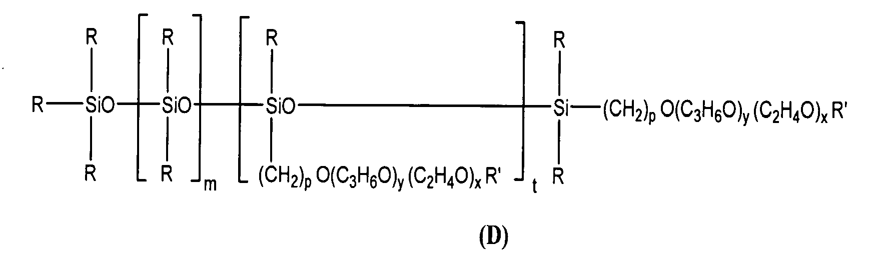 wobei die entzündungshemmenden Mittel ausgewählt sind aus der Gruppe, bestehend aus Hydrocortison, Hydrocortisonacetat, Prednisolon, Methylprednisolon, Prednisolonacetat, Prednisolonacetatpropionat, Dexamethason, Betamethason, Triamcinolon, Dexamethasonacetat, Betamethasonvaleriat, Triamcinolonacetonid, Aspirin, Acetaminophen, Glycolsalicylat, Mefenaminsäure, Flufenaminsäure, Indometacin, Diclofenac, Ketoprofen, Ibuprofen, Flurbiprofen, Fenbufen, Lufexamac, Piroxicam, Oxyphenbutazon, Mepirizol, Ibuprofenpiconol, Clidanac, Phenylbutazon, Naproxen, Glycyrrhetin, Glycyrrhizin, Glycyrrhetinsäure und den Salzen und Estern davon, Glycyrrhizinsäure und den Salzen und Estern davon, Azulen und Thymol.

2. Zusammensetzung gemäß Anspruch 1, welche weiterhin wenigstens einen Zucker umfasst.

3. Zusammensetzung gemäß Anspruch 2, wobei der Zucker ausgewählt ist aus der Gruppe, bestehend aus Monosacchariden, Oligosacchariden und Zuckeralkoholen.

4. Zusammensetzung gemäß einem der Ansprüche 2 und 3, wobei die Menge des Zuckers, welche in die Zusammensetzung formuliert wurde, 0,1 Gew.-% oder mehr beträgt.

5. Verwendung einer Zusammensetzung zur äußerlichen Behandlung der Haut, welche (i) 0,00001 bis 5,0 Gew.-% Vitamin A und (ii) wenigstens ein Hilfsmittel zur Verbesserung der Hautrauheit, das ausgewählt ist aus der Gruppe bestehend aus (a) 0,1 bis 20 % Polyoxyalkylen-modifizierten Organopolysiloxanen und (c) 0,0001 bis 5,0 % entzündungshemmenden Mitteln umfasst als Mittel zur Verbesserung oder Verminderung der Hautrauheit.

6. Verwendung gemäß Anspruch 5, wobei das Hilfsmittel zur Verbesserung der Hautrauheit wenigstens ein Polyoxyalkylen-modifiziertes Organopolysiloxan ist, das ausgewählt ist aus der Gruppe, bestehend aus den Verbindungen, welche durch die Formeln (A), (B), (C) und (D) unten dargestellt werden, wobei R für eine Alkylgruppe mit 1 bis 3 Kohlenstoffatomen oder eine Phenylgruppe steht, R' Wasserstoff oder eine Alkylgruppe mit 1 bis 12 Kohlenstoffatomen ist, p eine ganze Zahl von 1 bis 5 ist, m eine ganze Zahl von 5 bis 100 ist, n und x ganze Zahlen von 1 bis 50 sind, und t und y ganze Zahlen von 0 bis 50 sind:

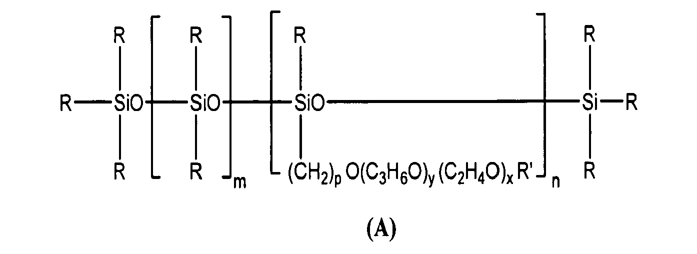

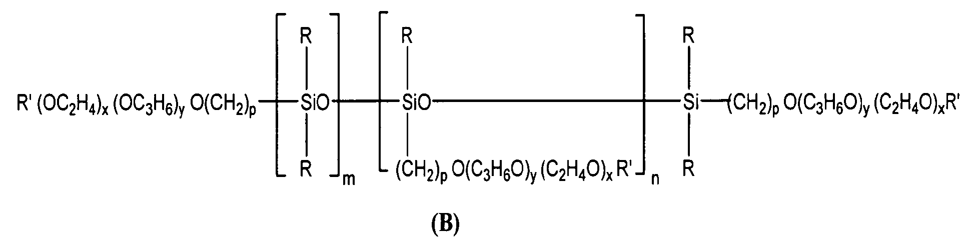

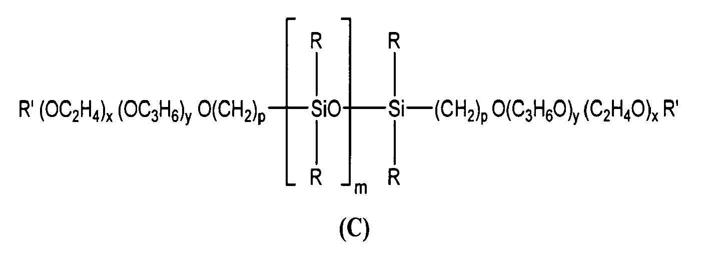

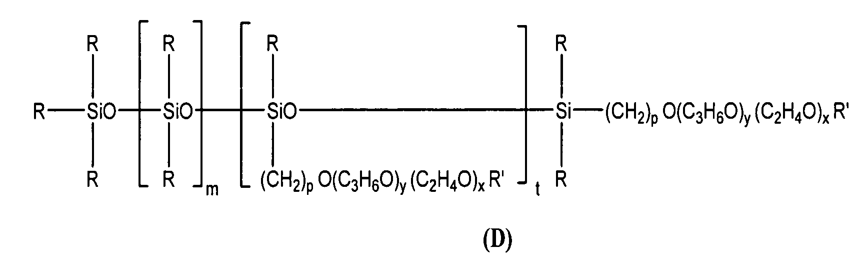

7. Verwendung gemäß Anspruch 5 oder 6, wobei das Hilfsmittel zur Verbesserung der Hautrauheit wenigstens ein entzündungshemmendes Mittel ist, das ausgewählt ist aus der Gruppe bestehend aus Hydrocortison, Hydrocortisonacetat, Prednisolon, Methylprednisolon, Prednisolonacetat, Prednisolonacetatpropionat, Dexamethason, Betamethason, Triamcinolon, Dexamethasonacetat, Betamethasonvaleriat, Triamcinolonacetonid, Aspirin, Salicylsäure, Acetaminophen, Methylsalicylat, Glycolsalicylat, Mefenaminsäure, Flufenaminsäure, Indometacin, Diclofenac, Ketoprofen, Ibuprofen, Flurbiprofen, Fenbufen, Lufexamac, Piroxicam, Oxyphenbutazon, Mepirizol, Ibuprofenpiconol, Clidanac, Phenylbutazon, Naproxen, Glycyrrhetin, Glycyrrhizin, Glycyrrhetinsäure und den Salzen und Estern davon, Glycyrrhizinsäure und den Salzen und Estern davon, Azulen, Campher, Thymol und Allantoin.

8. Verwendung gemäß einem der Ansprüche 5 bis 7, wobei die Zusammensetzung zur äußerlichen Behandlung der Haut weiterhin wenigstens einen Zucker, vorzugsweise einen Zucker, der ausgewählt ist aus der Gruppe bestehend aus Monosacchariden, Oligosacchariden und Zuckeralkoholen, umfasst.

## Revendications

1. Composition pour traitement cutané externe, comprenant (i) 0,00001 à 5,0 % en poids de vitamine A et (ii) au moins un adjuvant d'atténuation de rugosité cutanée choisi dans le groupe consistant en (a) 0,1 à 20 % en poids d'organopolysiloxanes à modification polyoxyalkylène et (c) 0,0001 à 5,0 % en poids d'agents anti-inflammatoires, lesdits organopolysiloxanes à modification polyoxyalkylène étant choisis dans le groupe consistant en les composés représentés par les formules (A), (B), (C) et (D) ci-dessous, dans lesquelles R représente un groupe alkyle ayant 1 à 3 atomes de carbone ou un groupe phényle, R' représente un atome d'hydrogène ou un groupe alkyle ayant 1 à 12 atomes de carbone, p représente un nombre entier de 1 à 5, m représente un nombre entier de 5 à 100, n et x représentent un nombre entier de 1 à 50 et t et y représentent un nombre entier de 0 à 50 : 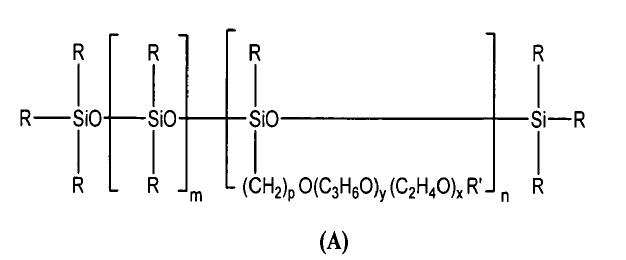 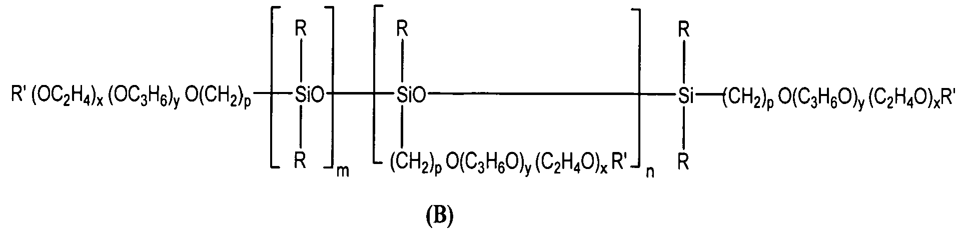 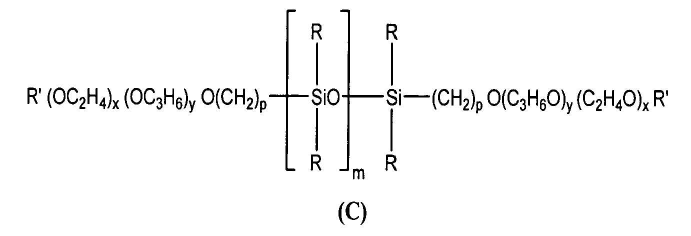 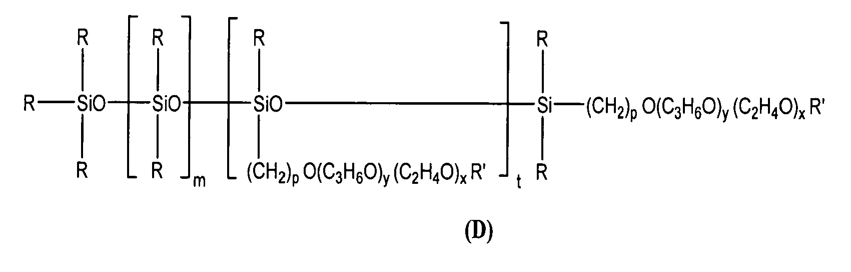 lesdits agents anti-inflammatoires étant choisis dans le groupe consistant en l'hydrocortisone, l'acétate d'hydrocortisone, la prednisolone, la méthylprednisolone, l'acétate de prednisolone, l'acétopropionate de prednisolone, la dexaméthasone, la bétaméthasone, la triamcinolone, l'acétate de dexaméthasone, le valérate de bétaméthasone, l'acétonide de triamcinolone, l'aspirine, l'acétaminophène, le salicylate de glycol, l'acide méfénamique, l'acide flufénamique, l'indométacine, le diclofénac, le kétoprofène, l'ibuprofène, le flurbiprofène, le fenbufène, le lufexamac, le piroxicam, l'oxyphenbutazone, le mépirizole, l'ibuprofène-piconol, le clidanac, la phénylbutazone, le naproxène, la glycyrrhétine, la glycyrrhizine, l'acide glycyrrhétique et ses sels et esters, l'acide glycyrrhzique et ses sels et esters, l'azulène et le thymol.

2. Composition suivant la revendication 1, qui comprend en outre au moins un sucre.

3. Composition suivant la revendication 2, dans laquelle le sucre est choisi dans le groupe consistant en des monosaccharides, des oligosaccharides et des sucre-alcools.

4. Composition suivant l'une des revendications 2 et 3, dans laquelle la quantité de sucre formulée dans la composition est égale ou supérieure à 0,1 % en poids.

5. Utilisation d'une composition de traitement cutané externe, comprenant (i) 0,00001 à 5,0 % en poids de vitamine A et (ii) au moins un adjuvant d'atténuation de rugosité cutanée choisi dans le groupe consistant en (a) 0,1 à 20 % d'organopolysiloxanes à modification polyoxyalkylène, et (c) 0,0001 à 5,0 % d'agents anti-inflammatoires en tant qu'agent pour atténuer ou réduire la rugosité de la peau.

6. Utilisation suivant la revendication 5, dans laquelle l'adjuvant d'atténuation de rugosité cutanée consiste en au moins un organopolysiloxane à modification polyoxyalkylène, choisi dans le groupe consistant en les composés représentés par les formules (A), (B), (C) et (D) ci-dessous, dans lesquelles R représente un groupe alkyle ayant 1 à 3 atomes de carbone ou un groupe phényle, R' représente un atome d'hydrogène ou un groupe alkyle ayant 1 à 12 atomes de carbone, p représente un nombre entier de 1 à 5, m représente un nombre entier de 5 à 100, n et x représentent un nombre entier de 1 à 50 et t et y représentent un nombre entier de 0 à 50 :

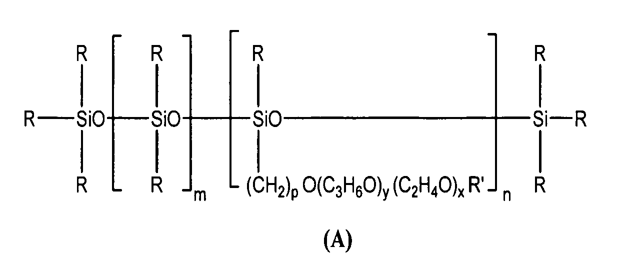

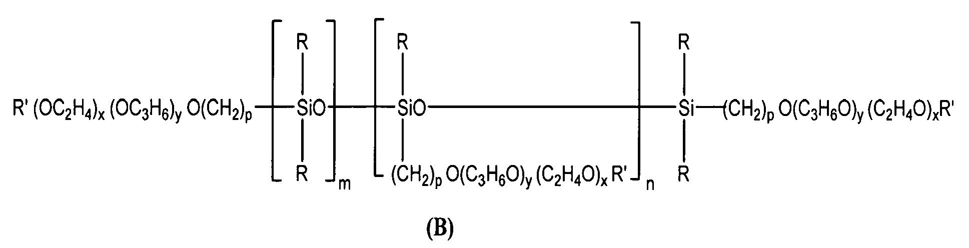

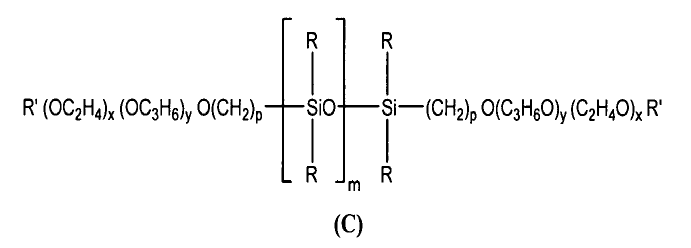

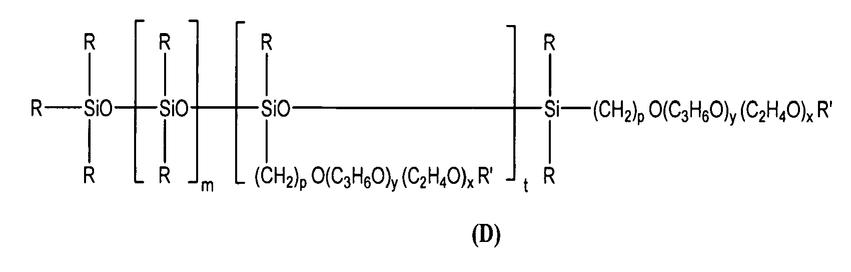

7. Utilisation suivant la revendication 5 ou 6, dans laquelle l'adjuvant d'atténuation de rugosité cutanée consiste en au moins un agent anti-inflammatoire choisi dans le groupe consistant en l'hydrocortisone, l'acétate d'hydrocortisone, la prednisolone, la méthylprednisolone, l'acétate de prednisolone, l'acétopropionate de prednisolone, la dexaméthasone, la bétaméthasone, la triamcinolone, l'acétate de dexaméthasone, le valérate de bétaméthasone, l'acétonide de triamcinolone, l'aspirine, l'acide salicylique, l'acétaminophène, le salicylate de méthyle, le salicylate de glycol, l'acide méfénamique, l'acide flufénamique, l'indométacine, le diclofénac, le kétoprofène, l'ibuprofène, le flurbiprofène, le fenbufène, le lufexamac, le piroxicam, l'oxyphenbutazone, le mépirizole, l'ibuprofène-piconol, le clidanac, la phénylbutazone, le naproxène, la glycyrrhétine, la glycyrrhizine, l'acide glycyrrhétique et ses sels et esters, l'acide glycyrrhizique et ses sels et esters, l'azulène, le camphre, le thymol et l'allantoïne.

8. Utilisation suivant la revendication 5, dans laquelle ladite composition de traitement cutané externe comprend en outre au moins un sucre, de préférence un sucre choisi dans le groupe consistant en des monosaccharides, des oligosaccharides et des sucre-alcools.
